(19) Europäisches Patentamt
European Patent Office
Office européen des brevets

(11) Veröffentlichungsnummer: **0 258 790 B1**

(12) # EUROPÄISCHE PATENTSCHRIFT

(45) Veröffentlichungstag der Patentschrift: **09.10.91**

(21) Anmeldenummer: **87112318.8**

(22) Anmeldetag: **25.08.87**

(51) Int. Cl.5: **C07D 307/68**, C07D 307/54, C07D 333/38, C07D 333/24, A01N 43/06, C07D 307/56, C07D 333/26

Die Akte enthält technische Angaben, die nach dem Eingang der Anmeldung eingereicht wurden und die nicht in dieser Patentschrift enthalten sind.

(54) **Furan- und Thiophencarbonsäurepropargylester.**

(30) Priorität: **30.08.86 DE 3629584**
**29.11.86 DE 3640878**

(43) Veröffentlichungstag der Anmeldung:
**09.03.88 Patentblatt 88/10**

(45) Bekanntmachung des Hinweises auf die Patenterteilung:
**09.10.91 Patentblatt 91/41**

(84) Benannte Vertragsstaaten:
**AT BE CH DE FR GB IT LI NL**

(56) Entgegenhaltungen:
**EP-A- 0 156 263**
**GB-A- 2 016 457**

(73) Patentinhaber: **BASF Aktiengesellschaft**
**Carl-Bosch-Strasse 38**
**W-6700 Ludwigshafen(DE)**

(72) Erfinder: **Neubauer, Hans-Juergen, Dr.**
**B 4,2**
**W-6800 Mannheim 1(DE)**
Erfinder: **Seppelt, Wolfgang, Dr.**
**Lucas-Cranach-Strasse 8**
**W-6712 Bobenheim-Roxheim(DE)**
Erfinder: **Buerstinghaus, Rainer, Dr.**
**Roentgenstrasse 38**
**W-6900 Heidelberg(DE)**
Erfinder: **Hofmeister, Peter, Dr.**
**Bernard-Humblot-Strasse 12**
**W-6730 Neustadt(DE)**
Erfinder: **Kuenast, Christoph, Dr.**
**Muehlstrasse 21**
**W-6701 Waldsee(DE)**

EP 0 258 790 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 100, Nr. 25, 18. Juni 1984, Seite 312, Zusammenfassung Nr. 206418j, Columbus, Ohio, US; G. KELLOVA et al.: "Antimicrobial effects of esters and amides of 3-(5-nitro-2-furyl)acrylic acid", & FOLIA MICROBIOL. (PRAGUE) 1984, 29(1), 23-34, & 11th COLLECTIVE INDEX CHEMICAL SUBSTANCES, 1987, Seiten 57041CS, 57042CS: unter 2-Propenoic acid, 3-(5-nitro-2-furanyl)-, 2-propynyl ester

CHEMICAL ABSTRACTS, Band 97, Nr. 17, 25. Oktober 1982, Seite 620, Zusammenfassung Nr. 144109t, Columbus, Ohio, US; A.I. LEVCHENKO et al.: "Polarography of furan-2-carboxylic acid esters", & KHIM. GETEROTSIKL. SOEDIN. 1982, (7), 890-2

**Beschreibung**

Die vorliegende Erfindung betrifft neue Furan- und Thiophencarbonsäurepropargylesterder allgemeinen Formeln Ia und Ib,

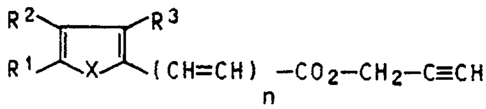

(Ia)

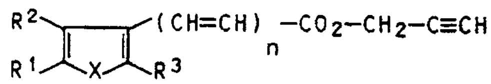

(Ib)

in denen die Substituenten und der Index folgende Bedeutung haben:

$R^1$, $R^2$, $R^3$: Wasserstoff, Chlor, Brom, $C_1$-$C_4$-Alkyl, $C_2$-$C_4$-Alkenyl, $C_1$-$C_4$-Halogenalkyl, $C_2$-$C_4$-Halogenalkenyl, Phenyl oder durch Halogen, $C_1$-$C_4$-Alkyl, $C_1$-$C_4$-Alkoxy substituiertes Phenyl,

X: Sauerstoff oder Schwefel,

n: die ganzen Zahlen 0 und 1,

wobei n nicht 0 bedeutet, wenn X für Sauerstoff und $R^1$, $R^2$ und$^3$ für Wasserstoff steht.

Für den Fall, daß X für Schwefel steht, bedeutet $R^1$, $R^2$ und $R^3$ zusätzlich Fluor, Cyano, $C_1$-$C_4$-Alkoxy, $C_1$-$C_4$-Halogenalkoxy, $C_2$-$C_4$-Alkenyloxy oder $C_2$-$C_4$-Halogenalkenyloxy.

Außerdem betrifft die Erfindung die Herstellung der Verbindungen Ia und Ib, Mittel zur Bekämpfung von Insekten, Spinnentieren und Nematoden, die die Verbindungen Ia bzw. Ib als Wirkstoffe enthalten, sowie ein Verfahren zur Bekämpfung von Insekten, Spinnentieren und Nematoden.

Aus EP-A-156,263, US-A-4,024,278 und US-A-3,996,380 sind Benzoesäurecarbonsäurepropargylester und andere Carbonsäurepropargylester als Schädlingsbekämpfungsmittel insbesondere gegen Insekten und Milben bekannt, jedoch enthalten diese Verbindungen keinen Furan- oder Thiophenring.

Aus Chemical Abstracts 100, 206418; sind Ester der 3-(5-Nitro-2-furyl)acrylsaüre - darunter der 2-Propinylester - mit antimikrobieller Wirkung bekannt.

Der Erfindung lag die Aufgabe zugrunde, neue Carbonsäurepropargylester mit modifiziertem Wirkungsprofil bereitzustellen.

Demgemäß wurden die eingangs definierten neuen Furan- und Thiophencarbonsäurepropargylester Ia und Ib sowie Verfahren zu deren Herstellung gefunden. Ferner wurde gefunden, daß sich die Verbindungen Ia und Ib zur Bekämpfung von Insekten, Spinnentieren und Nematoden eignen.

Die Verbindungen Ia und Ib sind nach folgenden Methoden erhältlich:

a) Die Umsetzung erfolgt zwischen einer Furan- oder Thiophencarbonsäure IIa bzw. IIb und Proparylalkohol (Houben-Weyl, Methoden der org. Chemie, Band VIII, S. 516ff, Georg-Thieme-Verlag, Stuttgart 1952), die durch Zusatz eines Katalysators wie Schwefelsäure, Halogenwasserstoff, Sulfonsäuren oder sauren Ionenaustauschern beschleunigt werden kann.

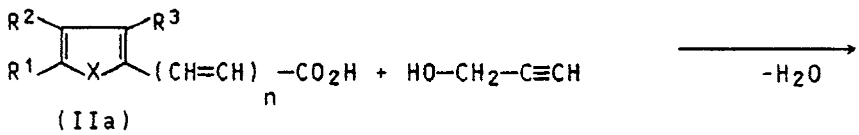


(IIa)

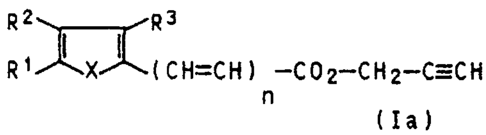

(Ia)

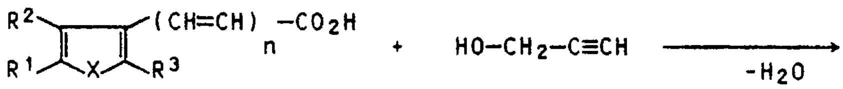


(IIb)

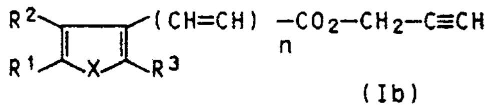

(Ib)

Das Veresterungsgleichgewicht kann in an sich bekannter Weise im gewünschten Sinne verschoben werden, indem man z.B. die Temperatur erhöht, das Wasser azeotrop abdestilliert oder an wasserentziehenden Mitteln bindet, wie Schwefelsäure, den Ester aus der Reaktionsmischung entfernt oder mit einem Überschuß einer der beiden Reaktionskomponenten arbeitet. Man arbeitet bei 0 bis 200 °C, vorzugsweise bei 20 bis 150 °C, und im Falle des azeotropen Abdestillierens bei der jeweiligen Übergangstemperatur des verwendeten Lösungsmittels.

Die Umsetzung wird zweckmäßigerweise in einem Lösungsmittel oder Verdünnungsmittel durchgeführt. Hierzu eignen sich beispielsweise aliphatische Kohlenwasserstoffe, wie n-Pentan, n-Hexan, das Hexan-Isomerengemisch und Petrolether; aromatische Kohlenwasserstoffe, wie Benzol, Toluol, die Xylole und deren Isomerengemische oder Benzin; chlorierte Kohlenwasserstoffe, wie Methylchlorid, Chloroform, Tetrachlorkohlenstoff und 1,2-Dichlorethan. Auch Gemische dieser Stoffe können als Lösungs- und Verdünnungsmittel verwendet werden.

b) Die Umsetzung erfolgt zwischen einem Furan- oder Thiophencarbonsäurehalogenid IIIa bzw. IIIb und Propargylalkohol in Gegenwart eines Säureacceptors (vgl. Houben-Weyl, Methoden der organischen Chemie, Band VIII, S. 543ff., Georg-Thieme-Verlag, Stuttgart 1952) im Temperaturbereich von (-30) bis 200 °C, vorzugsweise 0 bis 80 °C nach folgenden Reaktionsgleichungen:

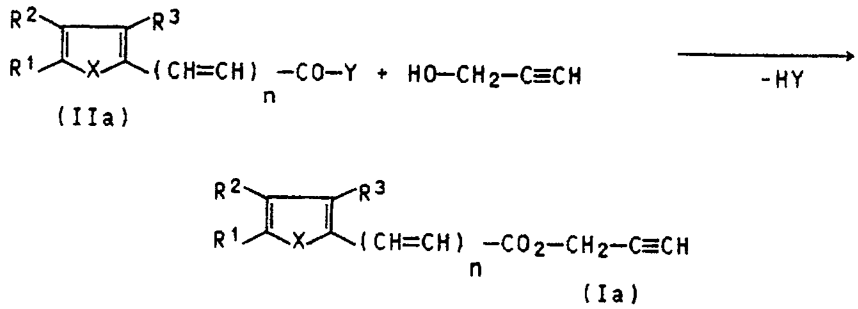


(IIa)

(Ia)

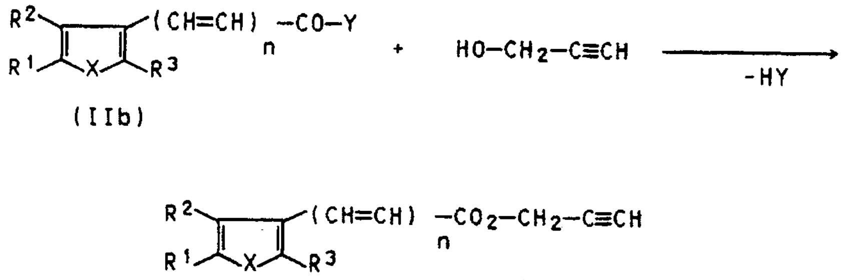


(IIb)

(Ib)

Y = Fluor, Chlor, Brom, Jod

Man setzt normalerweise mindestens equivalente Mengen einer Base zu, kann aber diese auch im Überschuß oder gegebenenfalls auch als Lösungsmittel verwenden. Als Basen eignen sich beispielsweise Hydroxide von Alkali- und Erdalkalimetallen, wie Natriumhydroxid, Kaliumhydroxid und Calciumhydroxid. Alkoholate von Alkali- und Erdalkalimetallen, wie Natriummethylat, Natriumethylat, Calciummethanolat oder Kalium-tert.-butylat; Alkali- oder Erdalkalimetallhydride, wie Natriumhydrid, Kaliumhydrid oder Calciumhydrid; Alkali- oder Erdalkalicarbonate, wie Natriumcarbonat, Kaliumcarbonat oder Calciumcarbonat; aliphatische Amine, wie Dimethylamin, Triethylamin oder Diisopropylamin; heterocyclische Amine, wie Piperidin, Piperazin oder Pyrrolidin; aromatische Amine, wie Pyridin oder Pyrrol und gegebenenfalls auch Alkyllithium-Verbindungen, wie n-Butyllithium.

Die Umsetzung wird zweckmäßigerweise in einem Lösungsmittel oder Verdünnungsmittel durchgeführt. Hierzu eignen sich beispielsweise aliphatische Kohlenwasserstoffe, wie n-Pentan, n-Hexan, das Hexan-Isomerengemisch und Petrolether; chlorierte Kohlenwasserstoffe, wie Methylenchlorid, Chloroform, Tetrachlorkohlenstoff, 1,2-Dichlorethan; aromatische Kohlenwasserstoffe, wie Benzol, Toluol, die Xylole und deren Isomerengemische, Benzin; chlorierte Aromaten, wie Chlorbenzol; Alkohole, wie Methanol, Ethanol, n-Propanol und Isopropanol; Ether, wie Diethyl-, Di-n-Butylether, Methyl-tert.-butylether, Tetrahydrofuran und Dioxan; Ketone, wie Aceton, Methylethylketon und Methylisopropylketon; Nitrile, wie Acetonitril und Propionitril; aprotische dipolare Lösungsmittel wie Dimethylformamid, Dimethylsulfoxid oder Pyridin. Auch Gemische dieser Stoffe können als Lösungs- und Verdünnungsmittel verwendet werden.

Die Furan- und Thiophencarbonsäuren und deren Säurehalogenide sind bekannt oder nach bekannten Methoden (s. Heterocyclic Compounds, Thiophene and its derivatives, Vol. 44, Part 1, S. 1ff, ed., by S. Gronowitz, John Wiley & Sons, New York 1985; Houben-Weyl a.a.O., S. 463ff) erhältlich. Propargylalkohol ist ein großtechnisches Produkt und kommerziell erhältlich.

Üblicherweise setzt man die Ausgangsstoffe in stöchiometrischem Verhältnis ein. Ein Überschuß des einen oder anderen kann in Einzelfällen aber durchaus vorteilhaft sein.

Die Umsetzung verläuft gewöhnlich oberhalb von 0 °C mit ausreichender Geschwindigkeit. Da sie teilweise unter Wärmeentwicklung verläuft, kann es von Vorteil sein, eine Kühlmöglichkeit vorzusehen.

Die erfindungsgemäßen Furan- und Thiophencarbonsäureester können außerdem noch nach praktisch allen bekannten Verfahren der Estersynthese hergestellt werden, beispielsweise durch Umsetzung von entsprechenden Carbonsäureanhydriden (vgl. Houben-Weyl a.a.O., S. 478) mit Propargylalkohol, durch Umsetzung von entsprechenden Carbonsäuresalzen mit Propargylhalogeniden oder durch Umesterungsreaktionen (vgl. Houben-Weyl a.a.O., S. 508 bis 628; C. Ferri, Reaktionen der organischen Synthese, S. 446 ff., Georg-Thieme-Verlag, Stuttgart 1978; S. Patai, The Chemistry of Carboxylic Acids and Esters, S. 505 ff., Interscience Publishers, London 1969).

Die erfindungsgemäßen Furan- und Thiophenderivate mit n = 1 können außerdem aus entsprechend substituierten Furan- und Thiophenaldehyden durch Kondensation mit dem geeignet aktivierten Essigsäureester hergestellt werden (vgl. J. Org. Chem. of VSSR (1970), 2309).

Die als Ausgangsmaterial benötigten Carbonsäuren IIa bzw. IIb sind entweder bekannt oder kommerziell erhältlich. Andernfalls lassen sie sich nach allgemein bekannten chemischen Verfahren herstellen (Comprehensive Heterocyclic Chemistry, Vol. 4, S. 531ff; A.R. Katritzky und C.W. Rees, Pergamon Press, 1984; Heterocyclic Compounds, Thiophene and its derivatives, Vol. 44, Part 1, S. 1 ff., ed. by S. Gronowitz, John Wiley & Sons, New York 1985; The Chemistry of Heterocyclic Compounds, Vol. 44, 3, Thiophene and its derivatives, S. 565 ff; John Wiley & Sons, 1986). Die in einigen der nachstehenden Beispiele verwendeten Carbonsäurechloride wurden z.T. nach Houben-Weyl a.a.0. S. 463 ff. aus den entsprechenden Carbonsäuren erhalten.

Im einzelnen haben die Substituenten und der Index folgende Bedeutungen:

$R^1$,$R^2$,$R^3$
- Wasserstoff
- Chlor, Brom
- $C_1$-$C_4$-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
- $C_2$-$C_4$-Alkenyl, wie Ethenyl, Prop-1-en-1-yl, Prop-2-en-1-yl, But-1-en-1-yl, But-2-en-1-yl und But-3-en-1-yl, bevorzugt Ethenyl,
- $C_1$-$C_4$-Halogenalkyl, bevorzugt $C_1$-$C_2$-Fluor- und Chloralkyl, wie Fluormethyl, Difluormethyl, Trifluormethyl, Chlormethyl, Dichlormethyl, Trichlormethyl, 2,2,2-Trifluorethyl und 2,2,2-Trichlorethyl,
- $C_2$-$C_4$-Halogenalkenyl, bevorzugt $C_2$-$C_4$-Fluor- und Chloralkenyl,
- Phenyl
- Halogenphenyl, bevorzugt Fluor- und Chlorphenyl, wie 2-Fluorphenyl, 4-Fluorphenyl, 2,4-Difluorphenyl, 2,4,6-Trifluormethyl, 2-Chlorphenyl, 4-Chlorphenyl, 2,4-Dichlorphenyl, 2,4,6-Trichlorphenyl und 2-Chlor-4-fluorphenyl,
- $C_7$-$C_{10}$-Alkylphenyl, wie 2-Methylphenyl, 4-Methylphenyl, 2,4-Dimethylphenyl, 2,4,6-Trimethylphenyl, 2-Ethylphenyl, 4-Ethylphenyl, 2,4-Diethylphenyl, 2-Ethyl-4-methylphenyl und 4-Ethyl-2-methylphenyl,
- $C_7$-$C_{10}$-Alkoxyphenyl, wie 2-Methoxyphenyl, 4-Methoxyphenyl, 2,4-Dimethoxyphenyl, 2,4,6-Trimethoxyphenyl, 2-Ethoxyphenyl, 4-Ethylphenyl, 2,4-Diethylphenyl, 2-Ethyl-4-methoxyphenyl und 4-Ethyl-2-methoxyphenyl,

X
- Sauerstoff
- Schwefel

n
- 0 und 1.

Für den Fall, daß X für Schwefel steht, haben die Substituenten $R^1$, $R^2$ und $R^3$ zusätzlich folgende Bedeutungen:

- Fluor
- Cyano
- $C_1$-$C_4$-Alkoxy, bevorzugt Methoxy, Ethoxy, n-Propoxy und iso-Propoxy,
- $C_1$-$C_4$-Halogenalkoxy, bevorzugt $C_1$-$C_2$-Fluor- und Chloralkoxy, wie Fluormethoxy, Difluormethoxy, Trifluormethoxy, Chlormethoxy, Dichlormethoxy, Trichlormethoxy, 2,2,2-Trifluorethoxy und 2,2,2-Trichlorethoxy,
- $C_2$-$C_4$-Alkenyloxy, wie Ethenyloxy, Prop-1-en-1-yloxy, Prop-2-en-1-yloxy, But-1-en-1-yloxy, But-2-en-1-yloxy und But-3-en-1-yloxy,
- $C_2$-$C_4$-Halogenalkenyloxy, bevorzugt $C_2$-$C_4$-Fluor- und Chloralkenyloxy.

Die Furan- und Thiophencarbonsäurepropargylester der allgemeinen Formeln Ia bzw. Ib sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Im Gegensatz zu den meisten bisher bekannten Wirkstoffen, die als Kontakt- oder Fraßgifte die Tiere töten, lähmen oder vertreiben, greifen die meisten der Verbindungen der Formel I in das hormonale System des tierischen Organismus ein. Bei Insekten wird beispielsweise die Umwandlung zur Imago, die Ablage von entwicklungsfähigen Eiern und die Entwicklung von abgelegten normalen Eiern gestört und dadurch die Generationsfolge unterbrochen. Für Wirbeltiere sind die erfindungsgemäßen Wirkstoffe praktisch ungiftig. Die meisten der Verbindungen der Formel I werden überdies leicht zu Stoffen abgebaut, die in der natürlichen Umgebung vorkommen und von Mikroorganismen weiter zerlegt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea ptiyocampa (Pinienprozessionsspinner), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagai (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-puncta (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Ortiorrhynchus sulcatus (Gefurchter Lappenrüßler), Otiorrhynchus ovatus (Erdbeerwurzelrüßler), Hylobius abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera) beispielsweise Lycoria pectoralis, Mayetiola destructor (Hessenfliege), Basineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hysocyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);

aus der Ordnung der Wanzen (Heteroptera) beispielsweise Nezara viridula (Grüne Reiswanze), Eurygaster integriceps (Asiatische Getreidewande), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlauf), Aphidula nasturtii (Kreuzdornblattlaus), Cerosipha gossypii (Gukrenblattlauf), Sappaphis mali (Rosige Apfellaus), Sappaphis mala (Mehlige Birnblattlaus), Dysaphis radicola (Mehige Apfelfaltenlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenalaus), Macrosiphon rosae (Große Rosenblattlaus), Megoura viciae (Wickenlaus), Schizoneura lanuginosa

(Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus); aus der Ordnung der Termiten (Isoptera) beispielsweise Reticulitermes lucifugus, Calotermes flavicollis, Leucotermes flavipes, Termes natalensis; aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blaberus giganteus (Riesenschabe).

Zur Klasse der Arachnoidea gehören Spinntentiere (Acarina) beispielsweise Ixodes ricinus (Holzbock), Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zysten bildende Nematoden, z.B. Globodera rostochiensis, Heterodera schachtii, Heterodera avenae, Heterodera glycinae, Heterodera trifolii, Stock- und Blattälchen, z.B. Ditylenchus dipsaci, Ditylenchus destructor, Pratylenchus neglectus, Pratylenchus penetrans, Pratylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z.B. in Form direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können als Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Aklylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 5 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter

Haftfähigkeit.

III. 10 Gew.-Teile der Verbindung Nr. 13 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 14 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 16 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %. Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,02 bis 10, vorzugsweise 0,08 bis 3,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Beispielsweise können folgende Mittel zugemischt werden: 1,2-Dibrom-3-chlorpropan, 1,3-Dichlorpropen, 1,3-Dichlorpropen + 1,2-Dichlorpropan, 1,2-Dibromethan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxo-lan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimi-dinyl-dimethylcarbamat, 2-Methyl-2-(methylthio)-propionaldehyd-O-(methylcarbamoyl)-oxim, S-Methyl-N[(methylcarbamoyl)-oxyl]-thio-acetimidat, Methyl-N',N'-dimethyl-N-[-(methylcarbamoyl)oxy]-1-thiooxamidat, N-(2-Methylchlorphenyl)-N',N'-dimethylformamidin, Tetrachlorthiophen, 1-(2,6-Difluor-benzoyl)-3-(4-chlorphenyl)-harnstoff, O,O-Dimethyl-O-(p-nitrophenyl)-phosphorthioat, O,O-Diethyl-O-(p-nitrophenyl)-phosphorthioat, O-Ethyl-O(p-nitrophenyl)-phenyl-phosphonothioat, O,O-Dimethyl-O-(3-methyl-4-nitrophenyl)-phosphorthioat, 0,0-Diethyl-O-(2,4-dichlorphenyl)-phosphorthioat, O-Ethyl-O-(2,4-dichlorphenyl)-phenyl-phosphonothioat, O,O-Dimethyl-O-(2,4,5-trichlorphenyl)-phosphorthioat, O-Ethyl-O-(2,4,5-trichlorphenyl)-ethyl-phosphorthioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-(p-methylsulfinyl-phenyl)-phosphorthioat, O-Ethyl-S-phenyl-ethyl-phosphonodithioat, O,O-Diethyl-[2-chlor-1-(2,4-dichlorphenyl)-vinyl]-phosphat, O,O-Dimethyl-[2-chlor-1-(2,4,5-trichlorphenyl)]-vinylphosphat, O,O-Dimethyl-S-(1'-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoyl-methyl)-phosphordithioat, O,O-Dimethyl-S-(N-methylcarbamoyl-methyl)-phosphorthioat, O,O-Dimethyl-S-(N-methoxyethyl-carbamoylmethyl)-phosphordithioat, O,O-Dimethyl-S-(N-formyl-N-methyl-carbamoyl-methyl)-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethylthiomethyl)-phosphordithioat, O,O-Diethyl-S[(p-chlorphenyltthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthio-ethyl)-phosphordithioat, O,O-Diethyl-S-(2-ethylsulfinylethyl)-phosphorthioat, O,O-Diethyl-thiophosphoryliminophenyl)-acetonitril, O,O-Diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O-[2-isopropyl-4-methylpyrimidinyl(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amidothioat, O,S-Dimethyl-N-acetyl-phosphoramidothioat, alpha-Hexachlorcyclohexan, 1,1-Di-(p-methoxyphenyl)-2,2,2-trichlor-ethan, 6,7,8,9,10,10-Hexa-chloro-1,5,5a,6,9,9a-hexahydro-6,9-methano-2,4,3-benzodioxathiepin-3-oxid, Pyrethrine, DL-2-Allyl-3-methyl-cyclopenten-(2)-on-(1)-yl-(4)-DL-cis,-trans-chrysanthemat, 5-Benzyl-furyl-(3)-methyl-DL-cis,-trans-chrysanthemat, 3-Phenoxybenzyl(±)-cis,-trans-2,2-dimethyl-3-(2,2-dichlorvinyl)-cyclopropancarboxylat, alpha-Cyano-3-phenoxybenzyl(±)-cis,-trans-2,2-dimethyl-3-(2,2-dichlorvinyl)-cyclopropancarboxylat, (s)-alpha-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,-trans-chrysanthemat, 2-Methyl-5-(2-propinyl)-3-furylmethyl-chrysanthemat, (alpha-Cyano-3-phenoxybenzyl)-alpha-isopropyl-4-chlorphenylacetat.

Herstellungsbeispiele von erfindungsgemäßen Furancarbonsäurepropargylestern Ia bzw. Ib

Verbindung Nr. 5 der nachfolgenden Tabelle 1:

Die Lösung von 1,8 g Propargylalkohol in 15 ml abs. Pyridin wird auf 5°C gekühlt und portionsweise mit 5,2 g 5-Chlorfuran-2-carbonsäurechlorid versetzt. Man läßt noch 12 h bei Raumtemperatur rühren, gießt in 100 ml Eiswasser und stellt mit konz. HCl auf pH 2 ein. Es wird dreimal mit Ether extrahiert und die vereinigten Extrakte mit 10prozentiger Natriumhydrogencarbonat-Lösung und Wasser gewaschen. Nach Trocknen über Natriumsulfat, Abziehen des Solvens i.Vak. und Umkristallisieren aus Methanol erhält man 2,2 g 5-Chlorfuran-2-carbonsäurepropargylester mit Schmelzpunkt Fp.: 58 bis 60°C.

Verbindung Nr. 14 der nachfolgenden Tabelle 1:

Die Lösung von 11,5 g 3-(5-Methylfuran-2-yl)-propensäurechlorid in 30 ml abs. Tetrahydrofuran wird unter Kühlung bei 0 bis 5°C mit 4,2 g Propargylalkohol und 20 ml Pyridin versetzt. Man läßt noch 12 Stunden bei Raumtemperatur rühren, filtriert vom ausgefallenen Hydrochlorid ab, engt i.Vak. ein und nimmt in 200 ml Essigester und 50 ml Wasser auf. Es wird dreimal mit Wasser gewaschen, über $Na_2SO_4$ getrocknet, das Solvens i.V. abgezogen und der Rückstand chromatographisch (Toluol/Essigester = 1/2) gereinigt. Man erhält 10,9 g 3-(5-Methylfuran-2-yl)-propensäurepropargylester, Brechungsindex $n_D^{26}$ = 1,578.

Verbindung Nr. 22 der nachfolgenden Tabelle 1:

6,8 g Propargylalkohol werden in 20 ml absolutem Pyridin gelöst. Nach Zugabe von 17,0 g 2,5-Dimethylfuran-3-carbonsäurechlorid bei höchstens 5°C läßt man 8 Stunden bei Raumtemperatur rühren. Man fügt 100 ml Diethylether zu, saugt ab und engt i.Vak. ein. Der Rückstand wird in 150 ml Essigester aufgenommen, mit Wasser ausgeschüttelt und die organische Phase über Natriumsulfat getrocknet. Nach Abziehen des Solvens i.V. wird aus n-Propanol/Wasser umkristallisiert. Man erhält 12,5 g 2,5-Dimethylfuran-3-carbonsäurepropargylester. Schmelzpunkt Fp.: 35 bis 37°C.

Tabelle 1

| Beispiel Nr. | Struktur entspr. Formel | n | $R^1$ | $R^2$ | $R^3$ | X | physik. Daten |
|---|---|---|---|---|---|---|---|
| 2 | Ia | 0 | $CH_3$ | H | H | O | $n_D^{22}$: 1,512 |
| 3 | Ia | 0 | H | $CH_3$ | H | O | |
| 4 | Ia | 0 | H | H | $CH_3$ | O | |
| 5 | Ia | 0 | Cl | H | H | O | Fp: 58 - 60°C |
| 6 | Ia | 0 | H | H | Cl | O | |
| 7 | Ia | 0 | Br | H | H | O | Fp: 87 - 89°C |
| 8 | Ia | 0 | H | H | Br | O | |
| 10 | Ia | 0 | $CH_2Cl$ | H | H | O | |
| 11 | Ia | 0 | 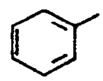 | H | H | O | |
| 12 | Ia | 0 | 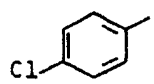 | H | H | O | |
| 13 | Ia | 1 | H | H | H | O | Fp: 44 - 45°C |
| 14 | Ia | 1 | $CH_3$ | H | H | O | $n_D^{26}$: 1,578 |
| 15 | Ia | 1 | H | H | $CH_3$ | O | |
| 16 | Ia | 1 | Cl | H | H | O | Fp: 54 - 55°C |
| 17 | Ia | 1 | H | H | Cl | O | |
| 18 | Ia | 1 | Br | H | H | O | |
| 19 | Ia | 1 | 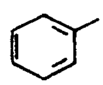 | H | H | O | |
| 20 | Ib | 0 | H | H | H | O | Oel |
| 21 | Ib | 0 | $CH_3$ | H | H | O | |

Tabelle 1 (Fortsetzung)

| Beispiel Nr. | Struktur entspr. Formel | n | $R^1$ | $R^2$ | $R^3$ | X | physik. Daten |
|---|---|---|---|---|---|---|---|
| 22 | Ib | 0 | $CH_3$ | H | $CH_3$ | O | Fp: 35 - 37°C |
| 23 | Ib | 0 | $CH_3$ | $CH_3$ | $CH_3$ | O | Fp: 52 - 54°C |
| 24 | Ib | 0 | H | H | $CH_3$ | O | $n_D^{22}$: 1,497 |
| 25 | Ib | 0 | Cl | H | H | O | |
| 26 | Ib | 0 | H | H | Cl | O | |
| 27 | Ib | 0 | 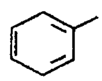 | H | H | O | |
| 28 | Ib | 1 | $CH_3$ | H | $CH_3$ | O | |
| 29 | Ib | 1 | H | H | H | O | |
| 30 | Ib | 1 | H | H | H | O | |

Herstellungsbeispiele von erfindungsgemäßen Thiophencarbonsäurepropargylestern Ia bzw. Ib

Verbindung Nr. 4 der nachstehenden Tabelle 2:

Die Lösung von 2,2 g Propargylalkohol in 35 ml absolutem Pyridin wird auf 5° C gekühlt und bei dieser Temperatur portionsweise mit 8,6 g 5-Bromthiophen-2-carbonsäurechlorid versetzt. Man läßt noch 8 Stunden bei Raumtemperatur rühren, gießt das Reaktionsgemisch in 100 ml Eiswasser und stellt mit konc. HCl auf pH 2 ein. Die wäßrige Phase wird dreimal mit Ether extrahiert und die vereinigten Etherextrakte mit 10prozentiger $NaHCO_3$-Lösung und Wasser gewaschen. Nach Trocknen über $Na_2SO_4$ und Abziehen des Solvens erhält man nach Umkristallisieren des Rückstandes aus n-Propanol/Wasser 4,2 g 5-Bromthiophen-2-carbonsäurepropargylester mit einem Schmelzpunkt von Fp.: 65 - 68° C.

Verbindung Nr. 25 der nachstehenden Tabelle 2:

Die Lösung von 6,2 g 3-(3-Methylthiophen-2-yl)-propensäure in 100 ml abs. DMF wird bei 10° C mit 0,9 g Natriumhydrid versetzt. Nach Beendigung der Wasserstoffentwicklung werden bei Raumtemperatur 4,9 g Propargylbromid zugetropft und anschließend 3 h auf 50° C geheizt. Zur Aufarbeitung wird der Reaktionsansatz auf Eiswasser gegossen, die wäßrige Phase dreimal mit Essigester extrahiert und die vereinigten organischen Extrakte über $Na_2SO_4$ getrocknet. Nach Abziehen des Solvens im Vakuum wird der verbleibende Rückstand aus Ethanol umkristallisiert. Man erhält so 3,9 g 3-(3-Methylthiophen-2-yl)-propensäurepropargylester vom Schmelzpunkt Fp.: 84 - 86° C.

Verbindung Nr. 45 der nachstehenden Tabelle 2:

4,7 g Propargylalkohol werden in 50 ml absolutem Pyridin gelöst. Nach Zugabe von 18,4 g 3-(2,3-Dichlorthiophen-4-yl)-propensäurechlorid bei maximal 0° C läßt man den Reaktionsansatz noch 12 Stunden bei Raumtemperatur rühren. Man fügt 50 ml Diethylether hinzu, saugt vom ausgefallenem Pyridinhydrochlorid ab und engt das Filtrat im Vakuum ein. Der Rückstand wird in 150 ml Essigester aufgenommen, mit Wasser geschüttelt und die organische Phase über $Na_2SO_4$ getrocknet. Nach Abziehen des Solvens im Vakuum wird der verbleibende Rückstand aus Methanol umkristallisiert. Man erhält 7,6 g 3-(2.3-Dichlorthiophen-4-yl)-propensäurepropargylester mit Schmelzpunkt Fp.: 84 - 86° C.

Tabelle 2

| Beispiel Nr. | Struktur entspr. Formel | n | $R^1$ | $R^2$ | $R^3$ | X | physik. Daten |
|---|---|---|---|---|---|---|---|
| 1 | Ia | 0 | H | H | H | S | visk. Öl |
| 2 | Ia | 0 | Cl | H | H | S | Fp.: 37-39°C |
| 4 | Ia | 0 | Br | H | H | S | Fp.: 65-68°C |
| 5 | Ia | 0 | Br | Br | H | S | Fp.: 60-63°C |
| 6 | Ia | 0 | H | H | $CH_3$ | S | $n_D^{23}$: 1.5512 |
| 7 | Ia | 0 | $H_3C$- | H | H | S | $n_D^{22}$: 1.5501 |
| 8 | Ia | 0 | H | Br | H | S | Fp.: 45-47°C |
| 9 | Ia | 0 | CN | H | H | S | |
| 10 | Ia | 0 | $CH_3$ | H | $CH_3$ | S | |
| 11 | Ia | 0 | $CH=CH_2$ | H | H | S | |
| 12 | Ia | 0 | $CH_2-CH_3$ | H | H | S | |
| 13 | Ia | 0 | 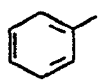 | H | H | S | |
| 14 | Ia | 0 | 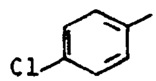 | H | H | S | |
| 15 | Ia | 0 | $CH_3O$ | H | H | S | |
| 16 | Ia | 0 | H | H | $CH_3O$ | S | |
| 17 | Ia | 0 | H | H | Cl | S | |
| 18 | Ia | 0 | Cl | H | Cl | S | |
| 19 | Ia | 0 | Cl | Cl | H | S | |
| 20 | Ia | 0 | Cl | Cl | Cl | S | |

Tabelle 2 (Fortsetzung)

| Beispiel Nr. | Struktur entspr. Formel | n | $R^1$ | $R^2$ | $R^3$ | X | physik. Daten |
|---|---|---|---|---|---|---|---|
| 21 | Ia | 1 | H | H | H | S | $n_D^{20}$: 1.6068 |
| 22 | Ia | 1 | H | Br | H | S | Fp.: 93-95$^0$C |
| 23 | Ia | 1 | Cl | H | H | S | Fp.: 51-53$^0$C |
| 24 | Ia | 1 | $CH_3$ | H | H | S | Fp.: 38-40$^0$C |
| 25 | Ia | 1 | H | H | $CH_3$ | S | Fp.: 74-78$^0$C |
| 27 | Ia | 1 | Br | H | H | S | Fp.: 68-70$^0$C |
| 28 | Ia | 1 | 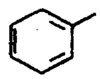 | H | H | S | |
| 29 | Ia | 1 | 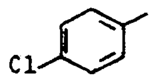 | H | H | S | |
| 30 | Ia | 1 | CN | H | H | S | |
| 31 | Ia | 1 | Cl | Cl | H | S | |
| 32 | Ib | 0 | H | H | H | S | $n_D^{22}$: 1.5494 |
| 33 | Ib | 0 | $CH_3$ | H | H | S | |
| 34 | Ib | 0 | Cl | Cl | H | S | Fp.: 48-50$^0$C |
| 35 | Ib | 0 | H | $CH_3$ | H | S | |
| 36 | Ib | 0 | H | Br | H | S | |
| 38 | Ib | 0 | $CH_3O$ | H | $CH_3$ | S | |
| 39 | Ib | 0 | Cl | H | Cl | S | |
| 40 | Ib | 0 | Br | H | H | S | |
| 41 | Ib | 0 | $CH_3$ | $CH_3$ | H | S | |
| 42 | Ib | 1 | H | H | H | S | $n_D^{22}$: 1.6053 |
| 43 | Ib | 1 | $CH_3$ | H | H | S | |
| 44 | Ib | 1 | $CH_3$ | H | $CH_3$ | S | |
| 45 | Ib | 1 | Cl | Cl | H | S | Fp.: 84-86$^0$C |
| 46 | Ib | 1 | Cl | H | H | S | |
| 47 | Ib | 1 | H | Br | H | S | |
| 48 | Ib | 1 | Br | Br | H | S | |

Anwendungsbeispiele

Beispiel A

Dysdercus intermedius (Baumwollwanze), Ovizide Wirkung

Stecketiketten werden am oberen Rand mit Klebestreifenstücken (ca. 0,8 cm) beklebt. 24 Stunden vor Versuchsbeginn werden die in einem Gefäß gesammelten Eier durch Eintauchen der Etiketten in das Gefäß

an dem Klebestreifenrand befestigt. Die Eier werden für ca. 5 Sekunden in die wäßrige Wirkstoffaufbereitung getaucht und auf Filterpapier abgetropft. Dabei sollen die Eier nicht auf das Papier gelegt werden. Die zurechtgeschnittenen Etiketten werden in Plastikpaletten gestellt, Klebestreifen nach oben. Eine halbe mit Wasser durchfeuchtete Watterolle wird in den Becher gelegt, um Austrocknung zu vermeiden, und die Palette wird mit einer Glasplatte abgedeckt. Nach ca. 8 Tagen wird bonitiert (bei der Kontrolle müssen die Larven geschlüpft sein).

In diesem Versuch erzielten die Verbindungen 2, 7 und 16 bei 0,02 Gew.% und die Verbindungen 1 und 5 bei 0,002 Gew.% eine 80 %ige Mortalität. Die mortale Dosis der Verbindungen 13 und 14 lag bei 0,002 Gew.%.

## Patentansprüche

**1.** Furan- und Thiophencarbonsäurepropargylester der allgemeinen Formeln Ia und Ib,

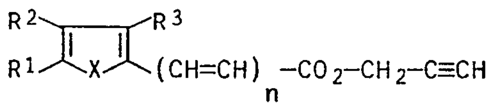

(Ia)

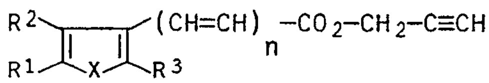

(Ib)

in denen die Substituenten und der Index folgende Bedeutung haben:

| | |
|---|---|
| $R^1$,$R^2$,$R^3$: | Wasserstoff, Chlor, Brom, $C_1$-$C_4$-Alkyl, $C_2$-$C_4$-Alkenyl, $C_1$-$C_4$-Halogenalkyl, $C_2$-$C_4$-Halogenalkenyl, Phenyl oder durch Halogen, $C_1$-$C_4$-Alkyl, $C_1$-$C_4$-Alkoxy substituiertes Phenyl, |
| X | Sauerstoff oder Schwefel, |
| n | die ganzen Zahlen 0 und 1, und |

für den Fall, daß X für Schwefel steht, $R^1$, $R^2$ und $R^3$ zusätzlich Fluor, Cyano, $C_1$-$C_4$-Alkoxy, $C_1$-$C_4$-Halogenalkoxy, $C_2$-$C_4$-Alkenyloxy oder $C_2$-$C_4$-Halogenalkenyloxy bedeutet,
wobei n nicht 0 bedeutet, wenn X für Sauerstoff und $R^1$, $R^2$ und $R^3$ für Wasserstoff steht.

**2.** Furan- und Thiophencarbonsäurepropargylester der nach Anspruch 1, in denen die Substituenten und der Index folgende Bedeutung haben:

| | |
|---|---|
| $R^1$ | Wasserstoff, Chlor, Brom, Methyl, Ethyl, Ethenyl, Chlormethyl, Phenyl oder p-Chlorphenyl, |
| $R^2$,$R^3$ | Wasserstoff, Chlor, Brom oder Methyl, |
| X | Sauerstoff oder Schwefel, |
| n | die ganzen Zahlen 0 und 1, und |

für den Fall, daß X für Schwefel steht, $R^1$ zusätzlich Cyano oder Methoxy und $R^3$ zusätzlich Methoxy bedeutet.

**3.** Furan- und Thiophencarbonsäurepropargylester nach Anspruch 1, in denen die Substituenten und der Index folgende Bedeutung haben:

| | |
|---|---|
| $R^1$ | Wasserstoff, Chlor, Brom oder Methyl, |
| $R^2$ | Wasserstoff, Chlor, Brom oder Methyl, |
| $R^3$ | Wasserstoff oder Methyl, |
| X | Sauerstoff oder Schwefel, und |
| n | die ganzen Zahlen 0 und 1. |

**4.** Verfahren zur Herstellung von Furan- und Thiophencarbonsäurepropargylestern gemaß Anspruch I, dadurch gekennzeichnet, daß man entweder

a) eine Furan- oder Thiophencarbonsäure der allgemeinen Formeln IIa oder IIb,

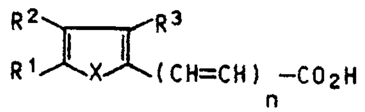

(IIa)

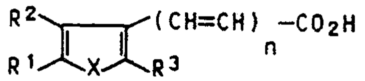

(IIb),

oder

b) ein Furan- oder Thiophencarbonsäurehalogenid der allgemeinen Formeln IIIa oder IIIb,

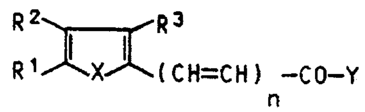

(IIIa)

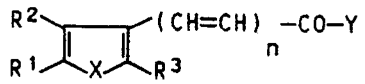

(IIIb),

in dem Y für Fluor, Chlor, Brom oder Jod steht, gegebenenfalls in Gegenwart eines Säureacceptors, mit Propargylalkohol in an sich bekannter Weise umsetzt.

5. Mittel zur Bekämpfung von Insekten, Spinnentieren und Nematoden, enthaltend einen Furan- oder Thiophencarbonsäurepropargylester gemäß Anspruch 1, in wirksamer Menge neben hierfür üblichen Trägerstoffen.

6. Mittel nach Anspruch 5, dadurch gekennzeichnet, daß es 0,1 bis 95 Gew.% eines Furan- oder Thiophencarbonsäurepropargylesters enthält.

7. Verfahren zur Bekämpfung von Insekten, Spinnentieren und Nematoden, dadurch gekennzeichnet, daß man die Insekten, Spinnentiere und Nematoden und/oder die von Insekten, Spinnentieren und Nematoden freizuhaltenden Flächen und/oder Räume mit einer für Insekten, Spinnentiere und Nematoden wirksamen Menge eines Furan- oder Thiophencarbonsäurepropargylesters gemäß Anspruch 1 behandelt.

## Claims

1. A propargyl furan- or thiophenecarboxylate of the formula Ia or Ib

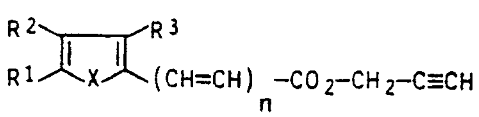

(Ia)

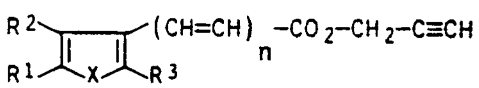

(Ib)

where $R^1$, $R^2$ and $R^3$ are each hydrogen, chlorine, bromine, $C_1$-$C_4$-alkyl, $C_2$-$C_4$-alkenyl, $C_1$-$C_4$-haloalkyl, $C_2$-$C_4$-haloalkenyl, phenyl which is unsubstituted or substituted by halogen, $C_1$-$C_4$-alkyl or $C_1$-$C_4$-alkory, X is oxygen or sulfur, n is the integer 0 or 1, and, if X is sulfur, $R^1$, $R^2$ and $R^3$ may furthermore be fluorine, cyano, $C_1$-$C_4$-alkoxy, $C_1$-$C_4$-haloalkoxy, $C_2$-$C_4$-alkenyloxy or $C_2$-$C_4$-haloalkenyloxy, n not being 0 if X is oxygen and $R^1$, $R^2$ and $R^3$ are each hydrogen.

2. A propargyl furan- or thiophenecarboxylate as claimed in claim 1, where $R^1$ is hydrogen, chlorine, bromine, methyl, ethyl, ethenyl, chloromethyl, phenyl or p-chlorophenyl, $R^2$ and $R^3$ are each hydrogen, chlorine, bromine or methyl, X is oxygen or sulfur, n is the integer 0 or 1, and, if X is sulfur, $R^1$ may furthermore be cyano or methoxy and $R^3$ may furthermore be methoxy.

3. A propargyl furan- or thiophenecarboxylate as claimed in claim 1, where $R^1$ and $R^2$ are each hydrogen, chlorine, bromine or methyl, $R^3$ is hydrogen or methyl, X is oxygen or sulfur and n is the integer 0 or 1.

4. A process for preparing a propargyl furan- or thiophenecarboxylate as claimed in claim 1, which comprises either

a) a furan- or thiophenecarboxylic acid of the formula IIa or IIb

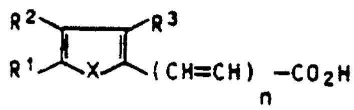

(IIa)

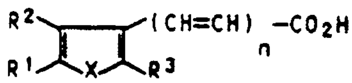

(IIb)

or

b) a furan- or thiophenecarbonyl halide of the formula IIIa or IIIb

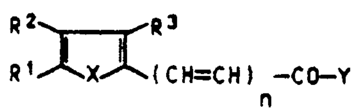

(IIIa)

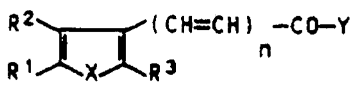

(IIIb)

where Y is fluorine, chlorine, bromine or iodine, being reacted, in the presence or absence of an acid acceptor, with propargyl alcohol in a conventional manner.

5. An agent for controlling insects, arachnids or nematodes, containing an effective amount of a propargyl furan- or thiophenecarboxylate as claimed in claim 1, in addition to conventional carriers.

6. An agent as claimed in claim 5, which contains from 0.1 to 95% by weight of a propargyl furan- or thiophenecarboxylate.

7. A method for controlling insects, arachnids and nematodes, wherein the insects, arachnids and nematodes and/or the areas and/or spaces to be kept free of insects, arachnids and nematodes are treated with an amount, effective for insects, arachnids and nematodes, of a propargyl furan- or thiophenecarboxylate as claimed in claim 1.

**Revendications**

1. Furanne- et thiophène-carboxylates de propargyle des formules générales Ia et Ib

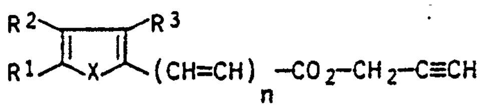

(Ia).

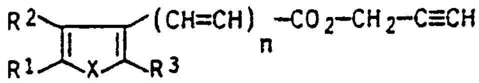

(Ib)

dans lesquelles les substituants et l'indice ont les significations suivantes :

$R^1$, $R^2$, $R^3$ : hydrogène, ohlore, brome, alkyle en $C_1$-$C_4$, alcényle en $C_2$-$C_4$, halogéno-alkyle en $C_1$-$C_4$, halogéno-alcényle en $C_2$-$C_4$, phényle ou phényle substitué par halogène, alkyle en $C_1$-$C_4$, alcoxy en $C_1$-$C_4$,

X oxygène ou soufre,
n les nombres entiers 0 et 1, et

dans le cas où X est mis pour soufre, $R^1$, $R^2$ et $R^3$ représentent en outre fluor, cyano, alcoxy en $C_1$-$C_4$, halogéno-alcoxy en $C_1$-$C_4$, alcényloxy en $C_2$-$C_4$ ou halogéno-alcényloxy en $C_2$-$C_4$, n ne représentant pas 0 lorsque X est mis pour oxygène et $R^1$, $R^2$ et $R^3$ pour hydrogène.

**2.** Furanne- et thiophène-carboxylates de propargyle selon la revendication 1, dans lesquels les substituants et l'indice ont les significations suivantes :

$R^1$ hydrogène, chlore, brome, méthyle, éthyle, éthényle, chlorométhyle, phényle ou p-chlorophnyle,
R2, $R^3$ hydrogène, chlore, brome ou méthyle,
X oxygène ou soufre,
n les nombres entiers 0 et 1, et

dans le cas où X est mis pour soufre, R1 représente additionnellement cyano ou méthoxy et $R^3$ additionnellement méthoxy.

**3.** Furanne- et thiophène-carboxylates de propargyle selon la revendication 1, dans lesquels les substituants et l'indice ont les significations suivantes :

$R^1$ hydrogène, chlore, brome ou méthyle,
R2 hydrogène, chlore, brome, ou méthyle,
R3 hydrogène ou méthyle,
X oxygène ou soufre, et
n les nombres entiers 0 et 1.

**4.** Procédé de préparation de furanne- et thiophène-carboxylates de propargyle selon la revendication 1, caractérisé par le fait que l'on fait réagir, éventuellement en présence d'un accepteur d'acide,
a) un acide furanne- ou thiophène-carboxylique des formules générales IIa ou IIb

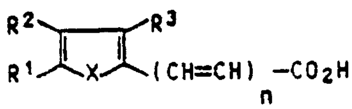

(IIa)

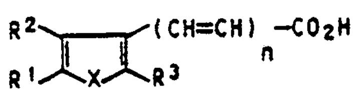

(IIb),

ou
b) un halogénure d'acide furanne- ou thiphène-carboxylique des formules générales IIIa ou IIIb

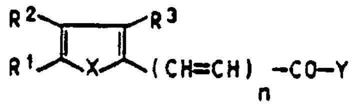

(IIIa)

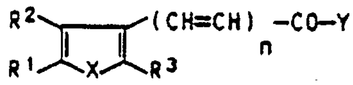

(IIIb),

dans lesquelles Y est mis pour fluor, chlore, brome ou iode, de manière en soi connue, avec de l'alcool propargylique.

5. Agents pour la lutte contre les insectes, arachnides et nématodes, contenant, outre des véhicules et excipients usuels, en une quantité efficace, un furanne- ou thiophène-carboxylate de propargyle selon la revendication 1.

6. Agent selon la revendication 5, caractérisé par le fait qu'il contient 0,1 à 95 % en poids d'un furanne-ou thiophène-carboxylate de propargyle.

7. Procédé de lutte contre les insectes, arachnides et nématodes, caractérisé par le fait que l'on traite les insectes, arachnides et nématodes et/ou les surfaces et/ou locaux à maintenir exempts d'insectes, d'arachnides et de nématodes, avec une quantité efficace vis-à-vis des insectes, arachnides et nématodes d'un furanne- ou thiophène-carboxylate de propargyle selon la revendication 1.